Europäisches Patentamt

European Patent Office

Office européen des brevets

(11) Veröffentlichungsnummer: **0 403 964**
**A2**

## (12) EUROPÄISCHE PATENTANMELDUNG

(21) Anmeldenummer: **90111289.6**

(22) Anmeldetag: **15.06.90**

(51) Int. Cl.5: **B01L 11/00, G01N 35/06**

(30) Priorität: **22.06.89 DE 3920476**

(43) Veröffentlichungstag der Anmeldung:
**27.12.90 Patentblatt 90/52**

(84) Benannte Vertragsstaaten:
**DE FR GB**

(71) Anmelder: **HOECHST AKTIENGESELLSCHAFT**
**Postfach 80 03 20**
**D-6230 Frankfurt am Main 80(DE)**

(72) Erfinder: **Schön, Klaus-Peter**
**Rathenauplatz 14**
**D-6200 Wiesbaden(DE)**

(54) **Dosier- und Füllgerät.**

(57) Das Dosier- und Füllgerät 1 besteht aus einer Dosiereinheit 10 für zu mischende Flüssigkeiten, einer Wägestation 2 mit einer Präzisionswaage 9 zum exakten Abwägen der einzelnen Flüssigkeitsmengen, einer Transportvorrichtung 23 für den Transport eines Meßglases 6, in welchem die Flüssigkeiten gemischt werden, zu einer Schwenkvorrichtung 30 für das Meßglas 6 und einer Flaschentransporteinheit 36, die einen Flaschenbehälter 37 für die Aufnahme von mehreren Flaschen 38 aufweist. In die einzelnen Flaschen 38 wird der Inhalt des Meßglases 6 eingefüllt.

FIG. 1

EP 0 403 964 A2

## DOSIER- UND FÜLLGERÄT

Die Erfindung betrifft ein Dosier- und Füllgerät für die Mengendosierung von Flüssigkeiten, die in einem Behälter zu einer Lösung mit bestimmten Mengenverhältnissen zusammengemischt und auf Flaschen gefüllt werden.

Die Mengendosierung von Flüssigkeiten ist insbesondere bei der Bestimmung des Biochemischen Sauerstoffbedarfs - BSB - von Gewässern anwendbar. Durch die Bestimmung des Biochemischen Sauerstoffbedars BSB, der ein summarischer Wirkungsparameter ist, kann eine Aussage über den Gehalt einer Substanz gemacht werden, die einem biologischen Abbau durch Mikroorganismen unterliegt. Der Biochemische Sauerstoffbedarf BSB ist definiert als diejenige Menge Sauerstoff, welche von Mikroorganismen benötigt wird, um die im Wasser enthaltenen organischen Substanzen bei 20 °C oxidativ abzubauen, z.B. innerhalb von fünf Tagen. Dieser Wert wird als $BSB_5$ bezeichnet. Seine Bestimmung wird in geschlossenen Flaschen vorgenommen, die die Lösung des Produkts in sauerstoffgesättigten, mikroorganismenhaltigem Wasser enthalten. Zu Beginn und zum Ende des Versuchs wird die Sauerstoffkonzentration gemessen. Für die Messungen sind in den Versuchsansätzen optimale physiologische Bedingungen, einschließlich der Versorgung mit mineralischen Nährstoffen, anzustreben. Da die Zusammensetzung und die stoffwechselphysiologischen Fähigkeiten der Mikroorga nismen den Versuchsablauf wesentlich mitbestimmen, kann im Einzelfall keine Vorhersage darüber getroffen werden, welchen zeitlichen Verlauf der Sauerstoffverbrauch nehmen wird. Der $BSB_5$ kann nicht aus einer Kurzzeitbestimmung durch Extrapolation ermittelt werden.

Laut DIN-Norm 38 409, Teil 51, ist das Meßverfahren auf alle Wässer anwendbar, die einen $BSB_5$ von mindestens 3 mg/l und von nicht mehr als 250 mg/l haben. Proben mit einem $BSB_5$ von mehr als 250 mg/l können nach Vorverdünnung untersucht werden.

Anteile der gegebenenfalls vorverdünnten Wasserprobe werden mit einem besonders vorbereiteten Verdünnungswasser entsprechend einer arithmetischen Reihe verdünnt. Die Ansätze werden für die vorgegebene Zeit, die in der Regel fünf Tage beträgt, bei 20 °C im Dunkeln mit einer Impflösung inkubiert. Aus dem Rückgang der Sauerstoffkonzentration in den Verdünnungen und im Verdünnungswasser wird der $BSB_5$ nach Maßgabe der Mischungsregel in geschlossener statistischer Auswertung ermittelt.

Um zu vermeiden, daß durch biochemische Oxidationen von Ammoniumstickstoff Ergebnisse erzielt werden, die sich schlecht reproduzieren und interpretieren lassen, wird den Proben ein Nitrifikationshemmstoff, im allgemeinen N-Allylthioharnstoff oder Trichlormethylpyridin, zugesetzt. Zur Kontrolle des Verdünnungswassers und des Impfstoffes wird der $BSB_5$ einer Standardlösung, der ge gebenenfalls der Nitrifikationshemmstoff zugesetzt ist, parallel bestimmt.

Weitere Einzelheiten des Meßverfahrens sind der voranstehend erwähnten DIN-Norm zu entnehmen.

Bisher war es üblich, für die $BSB_5$-Bestimmung die Impflösung, den N-Allylthioharnstoff und das Probewasser mittels Pipetten und das Verdünnungswasser durch Meßzylinder der zu untersuchenden Probe manuell zuzugeben.

Aufgabe der Erfindung ist es, ein Dosier- und Füllgerät für die Dosierung der Flüssigkeitsmengen zu schaffen, das eine sehr präzise und exakte Dosierung der Flüssigkeitsmengen ermöglicht und den gesamten Arbeitsablauf von Beginn der Dosierung an bis zum Entnehmen der gefüllten Probeflaschen automatisiert.

Diese Aufgabe wird erfindungsgemäß in der Weise gelöst, daß das Dosiergerät aus einer Wägestation, einer Dosiereinheit für die zu mischenden Flüssigkeiten, einer Transportvorrichtung für den Transport eines Meßglases, in dem die Flüssigkeiten gemischt sind, zu einer Schwenkvorrichtung für das Meßglas und einer Flaschentransporteinheit für Flaschen besteht, in die der Inhalt des Meßglases eingefüllt wird.

In Ausgestaltung der Erfindung umfaßt die Wägestation eine Präzisionswaage mit einem Zentrierring für das Meßglas, einen Sensor zum überwachen des Vorhandenseins des ließglases im Zentrierring und einen Fixierstab, in dessen Richtung die Ausgießnase des Meßglases ausgerichtet ist. In Weiterbildung der Erfindung verfährt ein Zylinder die Dosiereinheit horizontal aus einer Anfangsposition in eine Füllposition oberhalb des Meßglases im Zentrierring und umfaßt die Dosiereinheit ein Reduzierventil mit Manometer für Druckluft, zumindest zwei Fläschchen mit unterschiedlichen Lösungen, eine Probewasserflasche mit angeschlossener Pumpe zur Probenwasserdosierung, ein Dosierventil und zumindest zwei Zweiwegeventile.

Die weitere Ausgestaltung des Dosier- und Füllgeräts ergibt sich aus den Merkmalen der Patentansprüche 4 bis 14.

Die Erfindung wird im folgenden anhand eines zeichnerisch dargestellten Ausführungsbeispiels näher erläutert. Es zeigen:

Fig. 1 in schematischer Ansicht das Dosier- und Füllgerät nach der Erfindung, mit einem Meßglas in der Wägestation, und

Fig. 2 eine schematische Ansicht des Dosier- und Füllgeräts nach Fig. 1, mit dem angehobenen Meßglas oberhalb einer Aufnahmehalterung für das Meßglas.

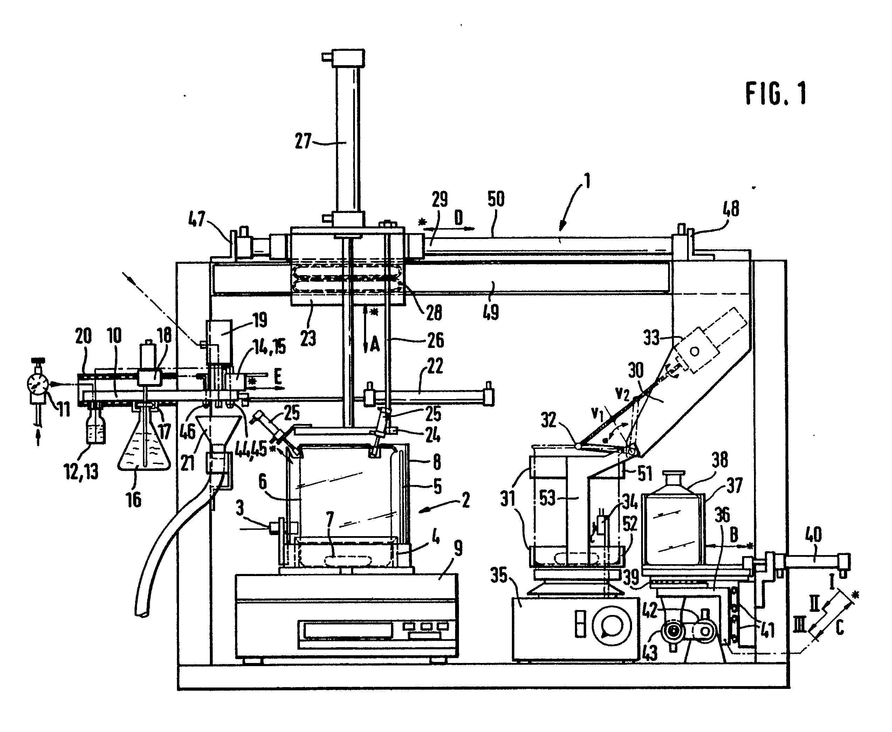

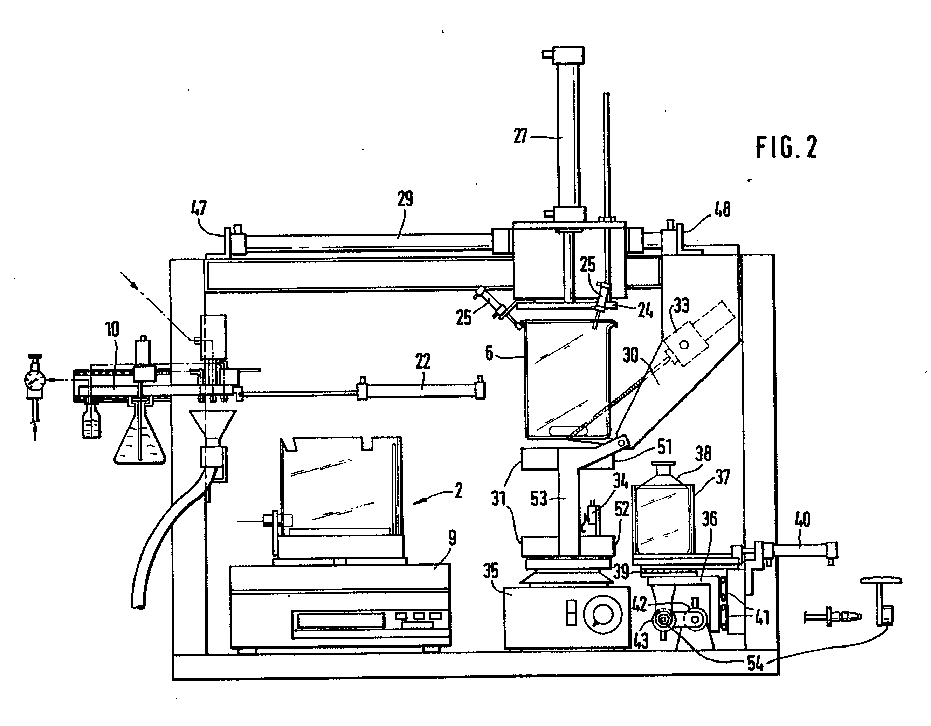

Das in den Figuren 1 und 2 dargestellte Dosiergerät 1 besteht aus einer Anzahl von Baugruppen, nämlich einer Wägestation 2, einer Dosiereinheit 10 für die zu mischenden Flüssigkeiten, einer Transportvorrichtung 23 für ein Meßglas 6, in welchem die Flüssigkeiten gemischt sind, einer Schwenkvorrichtung 30 für das Meßglas 6 und einer Flaschentransporteinheit 36 für Flaschen 38, in welche der Inhalt des Meßglases 6 eingefüllt wird.

Nach dem Einschalten einer speicherprogrammierbaren Steuerung des Dosier- und Füllgeräts 1 läuft ein Rückstellprogramm ab, bei dem alle Baugruppen in die durch einen Stern bei den Pfeilen A, B, C, D, E gekennzeichneten Anfangspositionen fahren. Dieses Rückstellprogramm läuft auch nach einer Notausschaltung des Dosier-und Füllgeräts 1, beispielsweise im Falle einer Überlastung, und dem Wiedereinschalten des Geräts ab.

Die Wägestation 2 umfaßt eine elektronische Präzisionswaage bekannter Bauart mit beispielsweise vier variabel wählbaren Gewichts- bzw. Dosiergrenzen. Auf der Präzisionswaage 9 befindet sich ein Zentrierring 4 für das Meßglas 6. Ein Sensor 3 ist horizontal ausgerichtet und überwacht das Vorhandensein des Meßglases 6 im Zentrierring 4. Seitlich von dem Meßglas 6 befindet sich ein vertikal stehender Fixierstab 5, in dessen Richtung eine Ausgießnase des Meßglases 6 ausgerichtet ist. In Figur 1 ist ein Magnetrührstäbchen 7 gezeigt, das durch einen Magnetrührer 35 in Drehungen versetzt wird, sobald das Meßglas 6 sich in einer Aufnahmehalterung 31 oberhalb dieses Magnetrührers 35 befindet. Durch die Drehungen des Magnetrührstäbchens 7 wird der Inhalt des Meßglases 6 gleichmäßig durchmischt.

Zu der Wägestation 2 gehört desweiteren ein zylindrischer Windschutz 8, der das Meßglas 6 und den Fixierstab 5 umschließt.

Oberhalb der Wägestation 2 befindet sich die Transportvorrichtung 23, die aus einer Greiferplatte 24 mit drei über den Plattenumfang verteilten Zylindern 25 besteht, die schräg zur horizontalen Greiferplatte angeordnet sind. Die Greiferplatte 24 wird durch einen Hubzylinder 27 vertikal angehoben und abgesenkt. Der Hubzylinder 27 ist an der Oberseite eines Schlittens 28 befestigt, der entlang zweier Schienen 49 von linearen Kugelführungen horizontal hin- und herverschiebbar ist. Für diese horizontale Bewegung der Transportvorrichtung ist ein kolbenstangenloser Transportzylinder 29 vorhanden, der sich entlang einer Achse 50 zwischen der Wägestation 2 und der Schwenkvorrichtung 30 bewegt.

Beim Einschalten des Dosier- und Füllgeräts 1 fährt die Greiferplatte 24 in Richtung des Doppelpfeils A nach unten, betätigt durch den Hubzylinder 27, und anschließend wieder in die durch einen Stern gekennzeichnete Anfangsposition zurück. In Figur 1 befindet sich das Meßglas 6 in der Wägestation 2, überwacht durch den Sensor 3, so daß das Rückstellprogramm blockiert ist. Das Meßglas 6 hat beispielsweise ein Volumen von einem Liter und ist in den Zentrierring 4 auf der Präzisionswaage 9 zwischen dem Windschutz 8 so gestellt, daß die Ausgießnase des Meßglases 6 auf den Fixierstab 5 zeigt. An der abgesenkten Greiferplatte 24 sind die Zylinder 25 bis unter einen Wulst des Meßglases 6 ausgefahren, so daß bei dem Anheben der Transportvorrichtung 23 durch die Zylinder 25 das Meßglas 6 mit angehoben wird. Sobald die Greiferplatte 24, betätigt durch den Hubzylinder 27, mit dem Meßglas 6 in die Anfangsposition fährt und die Transportvorrichtung 23 sich durch Betätigung des Transportzylinders 29 in Bewegung setzt, gelangt die Greiferplatte zusammen mit dem Meßglas in die in Figur 2 gezeigte Position oberhalb der Aufnahmehalterung 31 für das Meßglas 6 und des Magnetrührers 35.

Die Dosiereinheit 10 umfaßt ein Reduzierventil 11 mit einem Manometer für Druckluft, zumindest zwei Fläschchen von ca. 80 ml Inhalt, die unterschiedliche Lösungen enthalten, beispielsweise befindet sich in dem Fläschchen 12 eine Impflösung und in dem Fläschchen 13 N-Allylthioharnstoff, eine Probewasserflasche 16 mit angeschlossener Pumpe 18 zur probewasserdosierung, ein Dosierventil 19 und zumindest zwei Zweiwegeventile 14 und 15. Die beiden Fläschchen 12 und 13 sind an das Reduzierventil 11 angeschlossen. Jedes der beiden Zweiwegeventile 14 und 15 besitzt eine Düse mit einem Durchmesser von 1 mm für die Lösung bzw. für den N-Allylthioharnstoff. Die Probewasserflasche 16 hat zum Beispiel ein Volumen von ca. 600 ml und die Gestalt eines Erlenmeyer-Kolbens, der mittels einer Halterung 17 an der Unterseite der Dosiereinheit 10 befestigt ist. In den Figuren 1 und 2 sind die Fläschchen 12 und 13 in Richtung senkrecht zur Zeichenebene hintereinander angeordnet. Die an dem Reduzierventil 11 für Druckluft angeschlossenen Fläschchen 12 und 13 werden mit Druckluft von bis zu 0,25 bar beaufschlagt, wodurch die in den Flaschen befindlichen Lösungen über die Zweiwegeventile 14 und 15 zu den darin eingesetzten Düsen 44 und 45 geleitet werden, die, ebenso wie die Zweiwegeventile 14 und 15, in einer Richtung senkrecht zur Zeichenebene hintereinander angeordnet sind. Die Pumpe 18 ist mit einer weiteren Düse 46 in Verbindung, und alle drei Düsen 44, 45 und 46 sind nahe der Ausflußöffnung des Dosierventils 19 für Auffüll- bzw. Verdünnungswasser an der Unterseite der Dosiereinheit 10 angeordnet. Das Dosierventil 19 ist über eine strichpunktiert gezeichnete Leitung an einen nicht gezeigten Vorratsbehälter mit Verdünnungswasser angeschlossen. Unterhalb der Düsen 44, 45 und 46 sowie der Austrittsdüse des Dosierventils 19 ist ein Trichter 21 zum Auffangen der Flüssigkeiten, die vor dem eigentlichen Dosiervorgang zum Spülen durch die verschiedenen Leitungen fließen, angeordnet. Der Trichter 21 ist über einen Schlauch mit einem nicht dargestellten Auffangbehälter verbunden. Nach dem Einschalten des Dosier- und Füllgeräts 1 läuft die Pumpe 18 kurz an, um die Leitungen mit frischem Probewasser durchzuspülen. Danach fährt die Dosiereinheit 10 entlang zweier Teleskopschienen 20, die auf Kugellagern laufen, aus der Anfangs- in die Füllposition oberhalb des Meßglases 6. Für das horizontale Hin- und Herverfahren der Dosiereinheit 10 ist diese mit einem waagerecht gelagerten Zylinder 22 verbunden. Die Pumpe 18 für die Probewasserdosierung ist beispielsweise eine mit 24 V Gleichspannung betriebene Pumpe.

Sowie die Dosiereinheit 10 in der Füllposition über dem Meßglas 6 sich befindet, werden die Zweiwegeventile 14 und 15 kurz geöffnet, wobei die Öffnungszeit jedes der beiden Zweiwegeventile variabel einstellbar ist, so daß beispielsweise 0,2 mm Impflösung und 0,5 ml N-Allylthioharnstoff mit einem auf 0,04 bar eingestellten Druck durch die Düsen 44 und 45 in das Meßglas 6 dosiert werden. Die Präzisionswaage 9 wird von der selbstprogrammierbaren Steuerung auf Null gestellt, und die Pumpe 18 pumpt so lange Probewasser in das Meßglas 6 ein, bis die Präzsisionswaage 9 ihren ersten eingestellten Wägebereich von beispielsweise 250 ml erreicht hat. Daran anschließend wird die Pumpe 18 abgeschaltet und der zweite Wägebereich angesteuert, das Dosierventil 19 geöffnet und das Meßglas 6 mit Probewasser bis auf die gesamte Menge von einem Liter aufgefüllt. Die Dosiereinheit 10 fährt dann, betätigt durch den Zylinder 22, in ihre Anfangsposition zurück, die durch die Sternmarkierung an der einen Pfeilspitze des Doppelpfeils E angedeutet ist.

Die Transportvorrichtung 23 umfaßt desweiteren eine Zentrierstange 26, die mit einem Ende mit dem Schlitten 28 der linearen Kugelführungen lagefest verbunden ist und parallel zur Kolbenstange des Hubzylinders 27 für die Greiferplatte 24 verläuft. Das andere Ende der Zentrierstange 26 liegt an dem Umfangsrand der Greiferplatte 24 so an, daß ein Drehen der Greiferplatte um die Kolbenstange des Hubzylinders 27 blockiert ist.

Wie die Figuren 1 und 2 zeigen, befinden sich an den Enden der Achse 50, entlang welcher die Transportvorrichtung 23 horizontal hin- und herverfahrbar ist, Endanschläge 47, 48, welche die Bewegung der Transportvorrichtung in Positionen einerseits oberhalb der Wägestation 2 und andererseits oberhalb der Aufnahmehalterung 31 für das Meßglas 6 bzw. des Magnetrührers 35 begrenzen (vgl. Fig. 2).

Die Aufnahmehalterung 31 besteht aus zwei, miteinander durch Vertikalstreben 53 verbundenen Halteringen 51, 52, und es ist ferner ein Mikroschalter 34 zum Überwachen des Vorhandenseins eines Meßglases 6 im Bereich der Aufnahmehalterung 31 angebracht. Befindet sich ein Meßglas 6 in der Aufnahmehalterung, so ist der Mikroschalter 34 so geschaltet, daß er das Rückstellprogramm blokkiert. Anderenfalls, wenn kein Meßglas 6 in der Aufnahmehalterung vorhanden ist, läuft das Rückstellprogramm nach dem Einschalten des Dosier- und Füllgeräts 1 ab.

In Figur 2 befindet sich das ließglas 6 in der Aufnahmehalterung 31, nachdem die Greiferplatte 24 durch den Hubzylinder 27 abgesenkt wurde und die Zylinder 25 entlang dem Umfangsrand der Greiferplatte 24 zurückgefahren wurden. Danach fährt die Greiferplatte 24 hoch, und die Transportvorrichtung 23 bewegt sich in ihre Anfangsposition zurück.

Der Magnetrührer 35 ist in Betrieb und nach etwa zehn Sekunden Rührzeit, in welcher das Magnetrührstäbchen 7 im Meßglas 6 rotiert, läuft ein Antriebsmotor 33 der Schwenkvorrichtung 30 mit einer Geschwindigkeit $v_1$ an. Die Aufnahmehalterung 31 ist an der Schwenkvorrichtung 30 über eine dazu parallele Schwinge 32 angelenkt. Die Gewindespindel des Antriebsmotors 33 verschwenkt die Schwinge 32 und damit die aufnahmehalterung 31 mit dem Meßglas 6. Das in der Aufnahmehalterung befindliche Meßglas wird in eine schräge Ausgießposition oberhalb einer zu füllenden Flasche 38 in der Flaschentransporteinheit 36 hochgeschwenkt. Der Magnetrührer 35 schaltet dann aus, und bei einer Schräge von etwa 20° der Aufnahmehalterung 31 gegenüber der Horizontalen fährt die Flaschentransporteinheit 36, betätigt durch einen Zylinder 40, in eine Einfüllposition I vor, in welcher eine der drei Flaschen 38 sich unterhalb des hochgeschwenkten Meßglases 6 befindet.

Die Flaschentransporteinheit 36 wird mit Hilfe des Zylinders 40 entlang zweier Teleskopschienen 39, die auf Kugellagern laufen, horizontal verfahren. Unterhalb des Auflagetisches der Flaschentransporteinheit 36 sind zwei weitere Zylinder 42, 43 horizontal und im rechten Winkel zu dem Zylinder 40 angeordnet. Mit Hilfe dieser Zylinder 42, 43 wird die Flaschentransporteinheit 36 so verfahren, daß die leeren Flaschen 38 in die Einfüllpositionen I, II und III gebracht werden. Das Verstellen der Flaschentransporteinheit 36 erfolgt entlang zweier weiterer Teleskopschienen 41, die auf Kugellagern laufen und die übereinander angeordnet sind. Während der Geschwindigkeit $v_1$ der Schwenkvorrich-

tung 30 werden die erste und zweite Flasche 38 in die Einfüllpositionen III und II der Flaschentransporteinheit 36 durch den ersten Zylinder 42 gebracht und dort aufgefüllt. Ist die erste Flasche 38 bis zum Überlaufen gefüllt, fährt der Zylinder 42 die zweite Flasche 38 in ihre Einfüllposition, während die Schwenkvorrichtung weiterhin mit der Geschwindigkeit $v_1$ hochgeschwenkt wird. Sobald die zweite Flasche 38 vollgefüllt ist, wird über eine entsprechende Zeitsteuerung die Gewindespindel des Antriebsmotors 33 auf die langsamere Geschwindigkeit $v_2$ umgestellt und die dritte Flasche 38 durch den Zylinder 43 in ihre Einfüllposition I gebracht und aufgefüllt.

Die überlaufende Flüssigkeit wird in dem Flaschenbehälter 37 aufgefangen. Nach Beendigung des Füllvorgangs schwenkt die Schwenkvorrichtung 30 in ihre Anfangsposition zurück, und die Flaschentransporteinheit 36 wird gleichfalls in ihre Anfangsstellung zurückgefahren.

Die Flaschentransporteinheit 36 ist über eine Rastkupplung 54, die in Fig. 2 schematisch dargestellt ist, von dem zweiten Zylinder 43 zu trennen und vollständig aus dem Dosiergerät 1 herauszufahren, um den Flaschenbehälter 37 wechseln zu können. Wird das leere Meßglas 6 aus der Aufnahmehalterung 31 herausgenommen, so gibt der Mikroschalter 34 das Dosiergerät 1 zum neuerlichen Start frei.

Zur Begrenzung der Bewegung der Transportvorrichtung 23 entlang der Achse 50 in einer Position oberhalb der Wägestation 2 und oberhalb der Schwenkvorrichtung 30 bzw. der Aufnahmehalterung 31 schließen Endanschläge 47, 48 die Achse 50 ab.

Es ist offensichtlich, daß das Dosier- und Füllgerät 1 für das Mischen und exakte Zudosieren beliebiger Flüssigkeiten in einem Meßglas eingesetzt werden kann und keineswegs auf die Bestimmung des Biochemischen Sauerstoffbedarfs beschränkt ist.

**Ansprüche**

1. Dosier- und Füllgerät für die Mengendosierung von Flüssigkeiten, die in einem Behälter zu einer Lösung mit bestimmten Mengenverhältnissen zusammengemischt und auf Flaschen gefüllt werden, dadurch gekennzeichnet, daß das Dosiergerät (1) aus einer Wägestation (2), einer Dosiereinheit (10) für die zu mischenden Flüssigkeiten, einer Transportvorrichtung (23) für den Transport eines Meßglases (6), in dem die Flüssigkeiten gemischt sind, zu einer Schwenkvorrichtung (30) für das Meßglas (6) und einer Flaschentransporteinheit (36) für Flaschen (38) besteht, in die der Inhalt des Meßglases (6) eingefüllt wird.

2. Dosiergerät nach Anspruch 1, dadurch gekennzeichnet, daß die Wägestation (2) eine Präzisionswaage (9) mit einem Zentrierring (4) für das Meßglas (6), einen Sensor (3) zum überwachen des Vorhandenseins des Meßglases (6) im Zentrierring (4) und einen Fixierstab (5) umfaßt, in dessen Richtung die Ausgießnase des Meßglases (6) ausgerichtet ist.

3. Dosiergerät nach den Ansprüchen 1 und 2, dadurch gekennzeichnet, daß ein Zylinder (22) die Dosiereinheit (10) horizontal aus einer Anfangsposition in eine Füllposition oberhalb des Meßglases (6) im Zentrierring (4) verfährt und daß die Dosiereinheit (10) ein Reduzierventil (11) mit Manometer für Druckluft, zumindest zwei Fläschchen (12, 13) mit unterschiedlichen Lösungen, eine Probewasserflasche (16) mit angeschlossener Pumpe (18) zur Probewasserdosierung, ein Dosierventil (19) und zumindest zwei Zweiwegeventile (14, 15) umfaßt.

4. Dosiergerät nach Anspruch 3, dadurch gekennzeichnet, daß die Fläschchen (12, 13) einerseits an dem Reduzierventil (11) für Druckluft angeschlossen und mit Druckluft von bis zu 0,25 bar beaufschlagt sind, daß die Fläschchen andererseits über die Zweiwegeventile (14, 15) mit darin eingesetzten Düsen (44, 45) verbunden sind, daß die Pumpe (18) mit einer weiteren Düse (46) in Verbindung steht und daß die Düsen (44, 45, 46) nahe der Ausflußöffnung des Dosierventils (19) für Auffüll- bzw. Verdünnungswasser angeordnet sind.

5. Dosiergerät nach Anspruch 3, dadurch gekennzeichnet, daß die Dosiereinheit (10) entlang zweier Teleskopschienen (20), die auf Kugellagern laufen, aus der Anfangs- in die Füllposition und wieder zurück verfahrbar ist.

6. Dosierstation nach Anspruch 1, dadurch gekennzeichnet, daß die Transportvorrichtung (23) aus einer Greiferplatte (24) mit drei über den Plattenumfang verteilten Zylindern (25), einem die Greiferplatte vertikal anhebenden und absenkenden Hubzylinder (27), einem Schlitten (28) und zwei Schienen (49) linearer Kugelführungen und einem kolbenstangenlosen Transportzylinder (29) für die horizontale Bewegung der Transport vorrichtung entlang einer Achse (50) zwischen der Wägestation (2) und der Schwenkvorrichtung (30) besteht.

7. Dosierstation nach Anspruch 6, dadurch gekennzeichnet, daß ein Ende einer Zentrierstange (26) mit dem Schlitten (28) der linearen Kugelführungen lagefest verbunden ist und parallel zur Kolbenstange des Hubzylinders (27) für die Greiferplatte verläuft und daß das andere Ende der Zentrierstange an dem Umfangsrand der Greiferplatte (24) so anliegt, daß ein Drehen der Greiferplatte um die Kolbenstange blockiert ist.

8. Dosierstation nach Anspruch 6, dadurch gekennzeichnet, daß die Zylinder (25) schräg zur horizontalen Greiferplatte (24) angeordnet sind und mit ihren Kolbenstangen bis unter den Wulst des im Zentrierring (4) der Präzisionswaage (9) stehenden Meßglases (6) ausfahren, um dieses beim Hochfahren der Greiferplatte (24) aus dem Zentrierring (4) herauszuheben.

9. Dosierstation nach den Ansprüchen 1 und 6, dadurch gekennzeichnet, daß an den Enden der Achse (50) Endanschläge (47, 48) vorhanden sind, die die Bewegung der Transportvorrichtung (23) in Positionen oberhalb der Wägestation (2) und einer Aufnahmehalterung (31) für das Meßglas (6) bzw. eines Magnetrührers (35) begrenzen.

10. Dosierstation nach Anspruch 9, dadurch gekennzeichnet, daß auf dem Magnetrührer (35) die Aufnahmehalterung (31) für ein Meßglas (6) aufsitzt, die aus zwei Halteringen (51, 52), miteinander verbunden durch Vertikalstreben (53), besteht und daß ein Mikroschalter (34) zum Überwachen des Vorhandenseins des Meßglases (6) im Bereich der Aufnahmehalterung (31) angeordnet ist.

11. Dosiergerät nach den Ansprüchen 1 und 10, dadurch gekennzeichnet, daß die Aufnahmehalterung (31) an der Schwenkvorrichtung (30) angelenkt ist, die über eine dazu parallele Schwinge (32), betätigt durch eine Gewindespindel eines Antriebsmotors (33), verschwenkbar ist, um das in der Aufnahmehalterung befindliche Meßglas in eine schräge Ausgießposition oberhalb einer zu füllenden Flasche (38) in der Flaschentransporteinheit (36) zu verschwenken.

12. Dosiergerät nach den Ansprüchen 1 und 11, dadurch gekennzeichnet, daß die Flaschentransporteinheit (36) für die Aufnahme von Flaschen (38) einen Flaschenbehälter (37), der mit Hilfe eines Zylinders (40) entlang zweier Teleskopschienen (39) auf Kugellagern horizontal verfahrbar ist, aufweist, und daß zwei Zylinder (42, 43), die horizontal und im rechten Winkel zu dem einen Zylinder (40) angeordnet sind, der Reihe nach die leeren Flaschen (38) im Flaschenbehälter entlang zweier weiterer Teleskopschienen (41) auf Kugellagern in die Einfüllpositionen (III, II, I) unterhalb des schräg gestellen Meßglases (6) verfahren.

13. Dosiergerät nach den Ansprüchen 11 und 12, dadurch gekennzeichnet, daß die Gewindespindel des Antriebmotors (33) mit zwei Geschwindigkeiten ($v_1$, $v_2$) die Schwinge (32) verschwenkt, daß während des Verschwenkens mit der ersten Geschwindigkeit $v_1$, die größer als die zweite Geschwindigkeit $v_2$ ist, die erste und zweite Flasche (38) nacheinander in die Einfüllpisitionen (III, II) der Flaschentransporteinheit (36) durch den ersten Zylinder (42) verfahren und aufgefüllt werden und daß während des fortgesetzten Verschwenkens der Schwinge mit der Geschwindigkeit $v_2$ eine dritte Flasche in die Einfüllposition (I) durch den zweiten Zylinder (43) gebracht und aufgefüllt wird.

14. Dosiergerät nach Anspuch 12, dadurch gekennzeichnet, daß die Flaschentransporteinheit (36) über eine lösbare Rastkupplung (54) mit dem zweiten Zylinder (43) in Verbindung steht und nach dem Lösen der Rastkupplung zum Auswechseln der Flaschen (38) aus der Vorrichtung (1) herausziehbar ist.

FIG. 1

EP 0 403 964 A2

FIG.2

EP 0 403 964 A2